# EUROPEAN PATENT SPECIFICATION

(11) **EP 1 778 083 B1**
(45) Date of publication and mention of the grant of the patent: **26.05.2010**
(21) Application number: 05761725.0
(22) Date of filing: 12.07.2005
(51) Int. Cl.: A61B 5/08, A61B 5/113

(54) **METHOD AND SYSTEM FOR MONITORING VENTILATIONS**
VERFAHREN UND SYSTEM ZUR ÜBERWACHUNG VON BEATMUNGEN
PROCEDE ET SYSTEME DE SURVEILLANCE DE VENTILATIONS

(30) Priority: 15.07.2004 NO 20043033
(43) Date of publication of application: 02.05.2007
(73) Proprietor: Laerdal Medical AS, 4002 Stavanger (NO)
(72) Inventor: MYKLEBUST, Helge, N-4025 Stavanger (NO); NYSÆTHER, Jon, N-4041 Hafrsfjord (NO); STAVLAND, Mette, N-4027 Stavanger (NO); TELLNES, Geir, Inge, N-4048 Hafrsfjord (NO); EILEVSTJØNN, Joar, N-4323 Sandnes (NO)
(74) Representative: Thrane, Dag
(86) International application number: PCT/NO2005/000260
(87) International publication number: WO 2006/006871

(56) References cited:
- EP-A- 1 057 451
- EP-A- 1 079 310
- EP-A- 1 157 717
- WO-A-02/15836
- WO-A-2005/046431
- AASE S. ET AL.: "Compression Depth Estimation for CPR Quality Assessment Using DSP on Accelerometer Signals" IEEE TRANSACTIONS ON BIOMEDICAL ENGINEERING, vol. 49, no. 3, March 2002 (2002-03), pages 263-268, XP002367988

## Description

This method relates to a system and method for monitoring ventilations and compressions of a patient in cardiac arrest, especially as part of a CPR measurement and feedback system.

Quality of cardiopulmonary resuscitation, defined as chest compressions and ventilations, is essential for the outcome of cardiac arrest. Gallagher, Van Hoeyweghen and Wik, (Gallagher et al; JAMA 1995 Dec 27;274(24):1922-5. Van Hoeyweghen et al; Resuscitation 1993 Aug;26(1):47-52; Wik L, et al Resuscitation" 1994;28:195-203) respectively show that good quality CPR, performed by bystanders prior to the arrival of the ambulance personnel, can affect survival with a factor 3 - 4. But unfortunately, CPR is most often delivered with less than optimal quality, even by health care professionals according to a recent study published in JAMA (Wik et al. Quality of Cardiopulmonary Resuscitation During Out-of-Hospital Cardiac Arrest. Jama, January 19, 2005 - Vol 293, No 3). The most common failures are: Chest compressions are not delivered, ventilations are not delivered, chest compression depth is too shallow, chest compression rate is too high or too low, ventilation rate is too high or too low, or inflation time is too fast.

The international guidelines for resuscitation, issued by the American Heart Association, (Circulation 2000; 102 (suppl I)) express in detail how CPR should be delivered in order to be effective, and how CPR and defibrillation should be used together. Chest compression guidelines are uniform for all adult and older child patients: Depth should be 3 8-51mm, rate should be approximately 100/min, duty cycle should be about 50%, and rescuers should release pressure fully between compressions. Guidelines for ventilation are a bit more complicated. The amount of air to be delivered is first of all dependants upon whether supplemental oxygen is available. Titration could be based on detection of chest rise, or estimation of milliliters per kilo body weight (ml/kg), or estimation of milliliters. For instance, patients who are ventilated with > 40% oxygen should receive 6-7 ml/kg, but patient ventilated without oxygen should receive 10 ml/kg. But if your patient is hooked up to a transport ventilator, the amount is 10-15 ml/kg. Regarding ventilation rate, this depends on whether the patient has been intubated or not. Before intubation, rate is defined by the ratio of compressions to ventilation, which is currently set to 15 compressions to 2 ventilations. With this ratio, patients are expected to receive 6-8 ventilation per minute. After intubation, ventilation rate should now be 12-15 per minute. In addition, the guidelines express requirements for inflation time, which again is depending on volume. More recently, researchers have found that ventilation can have a detrimental effect on circulation. With hyperventilation during CPR, or when the proportion of time where intrathoracic pressure is elevated due to each ventilation is too large, it has been found that venous return is compromised and hence cardiac output is reduced.

The traditional method to ensure good quality CPR is by training. However, studies keep documenting that training does not provide skill retention. In other words, students may pass the test immediately followed by training, but skills deteriorate over time, and when the skill is needed, it is again suboptimal. This has led to the development of defibrillators, which is arranged to measure how CPR is performed, and to provide some kinds of feedback to the users. Groenke describe in US 6351671 an AED with force sensor, arranged to give feedback on chest compression rate and chest compression force, but not on ventilations. In EP 1215993 Myklebust disclose an AED with CPR sensors, where the performance characteristics of CPR is measured by the AED, where the AED then compare the CPR characteristics with the recommended characteristics according to the Guidelines, and where the AED then give correcting verbal feedback to the users. According to this invention, trans thoracic impedance vary with chest compressions and ventilations respectively. In particular, it is shown how chest compressions result in impedance changes having a morphology which is close to the morphology of chest compression movements, and how ventilations result in impedance changes having a morphology which is close to the ventilation volume waveform. One limitation with this invention is that the system will have difficulties in differentiating between compressions and ventilations, in particular when compression duty cycle is high and when ventilations inflation time is short. The system might also not be able to detect and characterize ventilations when compressions are ongoing.

In EP1079310, Myklebust describe a method to reduce artifacts in ECG caused by compressions and ventilations. This method is based on the availability of sensors that measure compressions and ventilations, that the signals from these sensors are used as reference signals for a digital adaptive filter. This filter can estimate the signal artifact and then subtract the estimated artifact from the original signal. This same principle can be applied to remove compression artifacts from impedance signals, such that the filtered impedance signal only reflect ventilation activity.

In EP1057451, Myklebust describe a sensor to measure chest compressions. This sensor is arranged with an accelerometer and a force activated switch. Part of the system is also means to estimate chest compression movement as a function of acceleration and signals from the force activated switch. One limitation by this sensor is that it does not provide means of reliably detecting that each chest compressions were completely released. One further limitation of this technology is that the precision of the system depends on what surface the patient is lying on. For instance, when tha patient is lying on a mattress, the senor on top of the chest will measure both the movement of the patient on the mattress and the compression of the chest. One more limitation has to do with those patients who has got a chest which is practically too stiff to be compressed down to the depth expressed by the guidelines. In this event, a feedback system connected to the chest compression sensor will continue to request more depth, although it is not practically possible.

Again in Norwegian patent application No 2004 3033, being the priority application of the present application, Myklebust further describe that a system arranged with a chest compression sensor and an adaptive filter, can be used to reduce artifacts from the impedance channel caused by chest compressions, in order to determine correct or wrongly placed intubation by comparing the magnitude of the impedance signal due to ventilation before intubation vs after intubation. Similar to EP1215993 , this system consist of a set of defibrillator electrodes attached to the patients' chest, which is connected to an external defibrillator, a chest compression sensor also connected to the defibrillator, a digital adaptive filter residing within the defibrillator and means to provide feedback to the user. A desired further application of this technology is to characterize ventilation performance while chest compressions are ongoing. Such characterization could be an estimate of tidal volume, an estimate of inflation time, an estimate of ventilations per minute, and an estimate of time where intra-thoracic pressure is elevated.

The present invention is now an extension of Norwegian patent application No 2004 3033 in order to characterize ventilation performance during ongoing chest compressions, to detect if every chest compression is fully released, to handle situations where the patient is lying on a compliant surface and to handle situations where the stiffness of the patient's chest does not allow compliance with the guidelines.

It is an object of this invention to provide a method and a system which can provide reliable indications of ventilation which also may be incorporated in existing life saving equipment, such as defibrillators.

It is known that impedance measurements may provide information about living tissue. This is usually performed by positioning electrodes on or in the body and applying a varying voltage or current through the electrodes. The impedance measurements with two or more electrodes are per se known to a person skilled in the art, and examples of such measuring systems are described in US 4540002, US 5807270 and WO 2004/049942. As described in the latter publication the impedance can be measured in chosen depths by using a number of electrodes.

### Summary of the invention

The proposed system provides an alternative method and system for montoring the ventilation of a patient by using a plurality of electrodes and a chest compression sensor attached to the patients thorax for measuring ventilation characteristics during resuscitation. Such electrodes and measuring systems are known from other application, like the solution described in EP 1215993 , measuring parameters for use in relation to a defibrillator and providing feedback to the user of the equipment thus helping him to perform the CPR, and EP 1057498, for measuring blood circulation using electrodes attached to the patients skin.

The abovementioned objects are obtained by a method and system as described in the accompanying claims.

When combined with other life saving equipment there is a problem in that such activities as chest compressions may affect the impedance, thus making the measurements more difficult to read. According to a preferred embodiment the invention comprises the use of adaptive filtering of the kind described in EP1079310 for removing these artifacts, the adaptive filtering also being per se known to a person skilled in the art.

The invention will be described below with reference to the accompanying drawings, illustrating the invention by way of examples.
- Figure 1: illustrates a patient on which the invention is used.
- Figure 2: illustrates the system according to a preferred embodiment of the invention.
- Figure 3: illustrates the signal filtering according to a preferred embodiment of the invention, where the upper trace is the filtered impedance signal indicating ventilation activity, the middle trace is the unfiltered impedance signal, and the bottom trace is the compression depth signal
- Figure 4: Upper trace is the filtered impedance signal indicating ventilation inactivity and that indication of ventilation is no longer present on the right side of the picture, suggesting esophageal intubation. Lower trace is the compression depth signal.
- Figure 5: illustrates the signal filtering according to a preferred embodiment of the invention, where the upper trace is the filtered impedance signal indicating ventilation activity after successful intubation, the middle trace is the unfiltered impedance signal, and the bottom trace is the compression depth signal
- Figure 6: illustrates the measured force signal and related time windows
- Figure 7: illustrates the individual components of the impedance signal
- Figure 8: illustrates the features which is used to characterize an impedance change as a ventilation event.
- Figure 9: illustrate the structure of one digital adaptive filter arrangement according to a preferred embodiment of the invention.

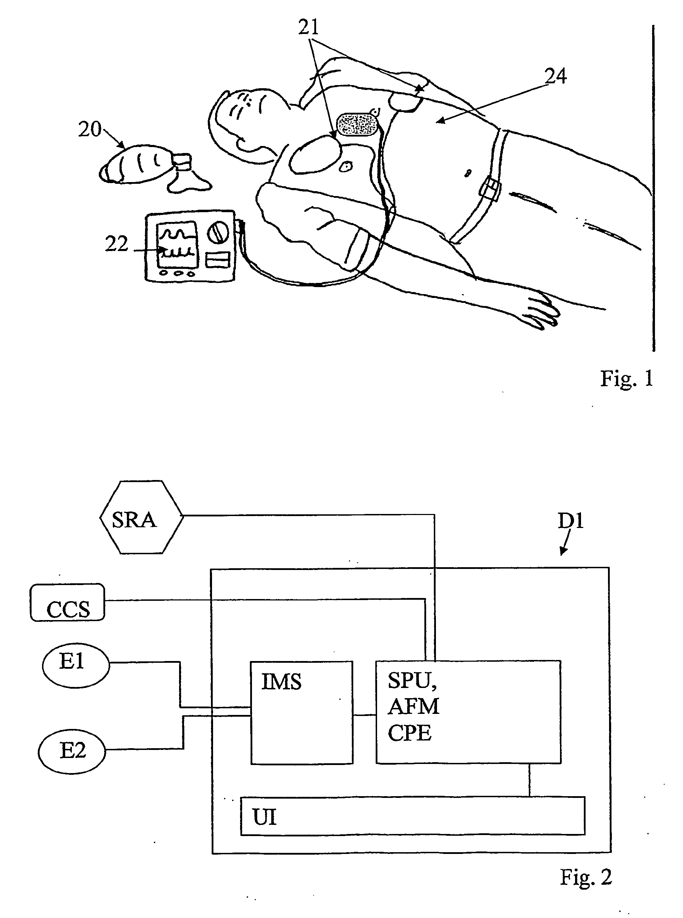

The system according to the invention illustrated in figure 1 comprises at least two electrodes 21 attached to the patient's thorax 24, an impedance measurement system connected to the electrodes, a microcomputer connected to the impedance measurement system and a display unit connected to the microcomputer, all arranged within a device 22.

The processing unit 22 illustrated may be provided with visual or acoustic means for providing feedback to the user, e.g. instructing the user in the same way as described in EP 1215993 to retract or adjust the position of the tube or simply by triggering an acoustic warning signal.

According to a preferred embodiment of the invention the system according to the invention is incorporated in an external defibrillator. The advantage of such an embodiment is cost, space and time saving. A defibrillator is normally arranged with a plurality of electrodes attached to the patient's thorax, an impedance measuring system connected to the electrodes, a microcomputer connected to the impedance measurement system and a display unit connected to the microcomputer. Hence, integrating the technology is a matter of design.

Figure 2 shows a system overview with a defibrillator D1 being coupled to a chest compression sensor (CCS) and defibrillator electrodes E1, E2. The defibrillator comprises an impedance measurement system (IMS), a signal processing unit (SPU), including an adaptive Filter Method (AFM), CPR parameter extractor (CPE) and a user Interface (UI) for communicating with the user.

The chest compression sensor CCS consists of a housing in which one accelerometer and one force transducer are arranged together and connected to a local microcontroller.

The microcontroller is arranged with signal inputs to read force and acceleration signals, and signal outputs to control the accelerometer and force transducer. Test software is arranged such that the microcontroller can test the integrity of the system. Furthermore, the microcontroller is arranged with communication means, in order to provide data to the SPU and defibrillator. A graphical illustration is arranged on top of the sensor to illustrate its placement. Underneath, the sensor is arranged with means to adhere the sensor to the patient's skin.

The impedance measurement system IMS is connected to standard defibrillators electrodes. Preferably, the IMS uses a near constant alternating current, which is applied between the electrodes, to generate a voltage which becomes proportional to the magnitude of the impedance between the electrodes. The voltage is subject to measurement and filtering, in order to output an impedance signal. This measurement system has preferably got a resolution of 10 milliohms, and a frequency response of DC to approximately 50 Hz. Further details on how such a IMS can be arranged can be found in EP1215993, EP1057498). The output impedance signal is connected to the SPU.

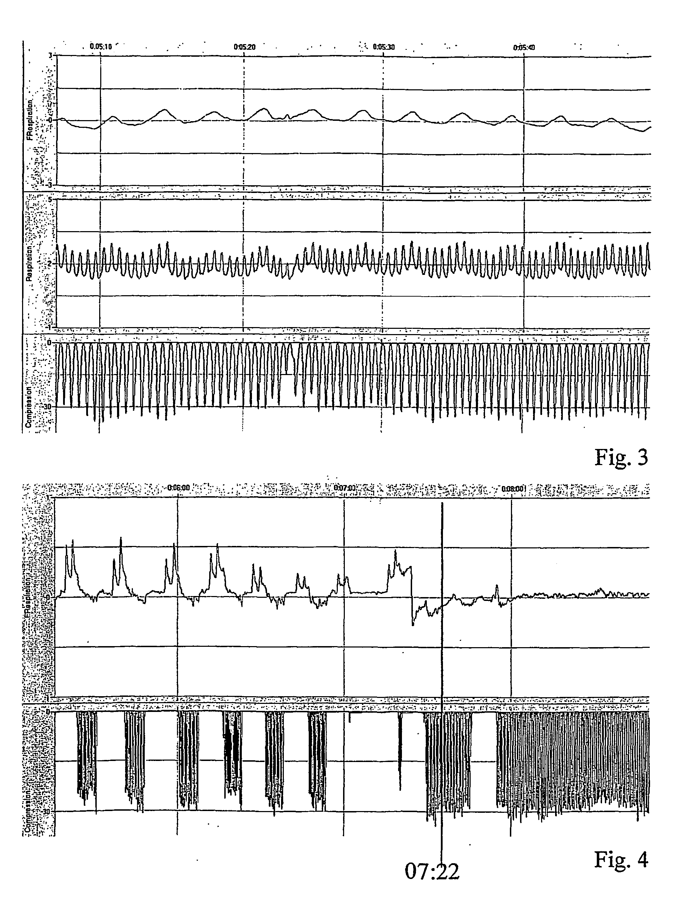

Typical impedance variations when the patient is ventilated with a standard bag is 0.5 - 1.0 ohm, but some types of bodies deviates from this. Large bodies may be as low as 0.50hm, and small bodies more than 1,5 Ohm. Mechanical conditions related to lungs and chest may also give deviations, this including lung edema, lung cancer, trauma, drowning, foreign elements, etc. Figures 3, 4 and 5 illustrate examples of impedance variations which will be described more in detail below.

Typical design characteristics of the system: Electrodes are arranged on the thorax preferably over both lungs. The impedance measurement system has a resolution of 10 milliohm. Dynamic range of the measurement system is from 0 ohm to 250 ohm, when typical defibrillator electrodes are used. The impedance measurement system typically uses a near constant AC current of 0.1 to 3 mA, and the AC frequency is typically in the range of 30kHz to 200 kHz.

The computer can be arranged to memorize the impedance changes in different parts of the measurements, e.g. to see whether an intubation is successful. The computer can also be arranged to fully guide the user trough the steps of ventilation and CPR using audible prompts, text, pictograms, video or any combination of audible and visible guiding.

One other embodiment is a standalone unit arranged to just facilitate intubation support and ventilation support. Such a standalone unit can be further expanded to facilitate CPR feedback as described in EP 1157717 , using a chest compressions sensor as in EP 1057451 and other sensors that describe how CPR is performed and how the patient responds to the CPR. Patient response to CPR can be determined using ECG analysis EP 1215993 and end tidal CO2 measurements.

Confirmation of tube placement can also be done during chest compressions, provided that the system is expanded with a digital adaptive filter which principle is detailed below and in EP1079310, which is included here by way of example. According to this invention however, it is the compression artifact on the impedance signal that is filtered, using signal from a chest compression sensor as reference input. This filter may take into account different types of measurements done during chest compressions, such as the measured movements of the chest, applied pressure or acceleration of a sensor positioned on the chest.

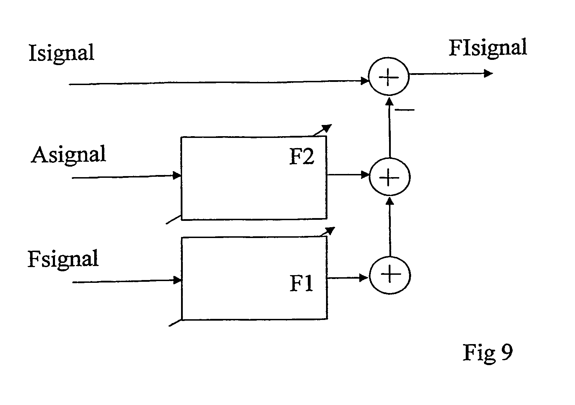

Figure 9 illustrate the structure of one digital adaptive filter arrangement for this purpose. The discrete Force signal samples Fsignal is fed into a first filter block F1, whose filter coefficients can be adjusted for each incoming sample value. The discrete Acceleration signal sample Asignal is fed into a second filter block F2, whose filter coefficients also can be adjusted for each incoming sample value. The filter outputs are summed, and then subtracted to the discrete Impedance signal sample Isignal, which result in the discrete filtered impedance signal IF signal.

By measuring these parameters the effects of the chest compressions may be filtered out from the impedance signal so as to obtain control of the intubation by calculating the maximum correlation between a reference signal, e.g. a signal obtained without chest compressions, and the measured signal.

Figure 3 show traces of digital adaptive filtered impedance waveform (top), the corresponding impedance raw signal (middle) and the chest compression depth waveform (bottom). The ventilations are evident in the top trace. Chest compressions cause artifacts in the impedance raw signal, which is effectively removed by the digital adaptive filter.

Figure 4 show traces of impedance changes (top) and chest compressions depth (bottom) before and after an intubation performed for applying ventilation to the patient The two ventilations in between series of 15 .compressions are evident in the beginning of the figure. Here ventilations were delivered using face mask and bag. Intubation was performed at time 07:22, and after then the impedance signal disappears. This indicates that the tube was misplaced and should be adjusted or removed.

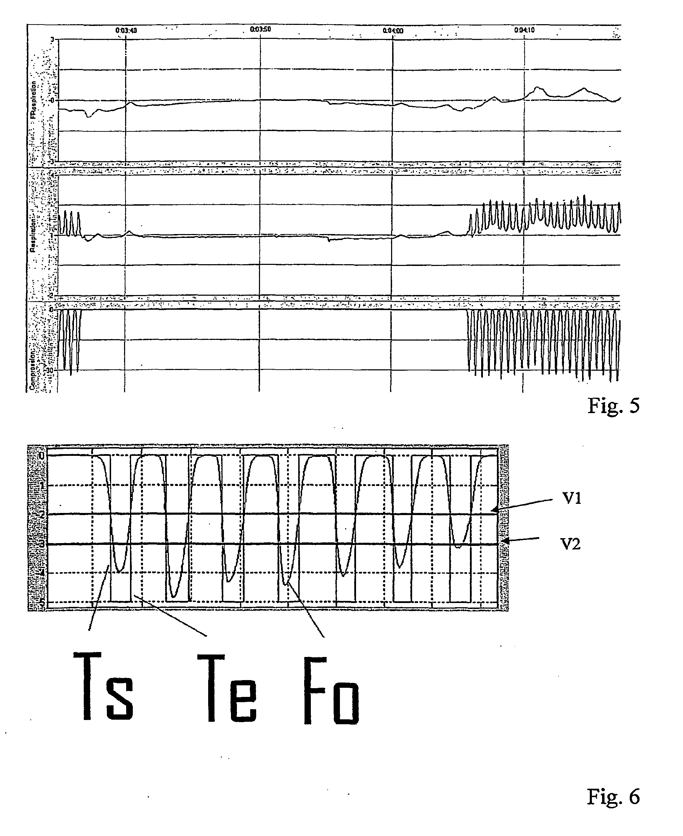

Figure 5 shows an example of successful intubation: The top and middle trace show two ventilations followed by a long pause when intubation is performed. Then ventilations and chest compressions resume. We can see that the magnitude of the impedance signal after intubation is greater than before.

Although the invention described above is mainly aimed at automated recognition and comparing of the stored impedance data the comparison may be performed manually by inspecting the curves, such as illustrated in figures 3-5, during the operation. The stored data is then kept sufficiently long to enable the user to see excerpts of both the data with and without the inserted tube.

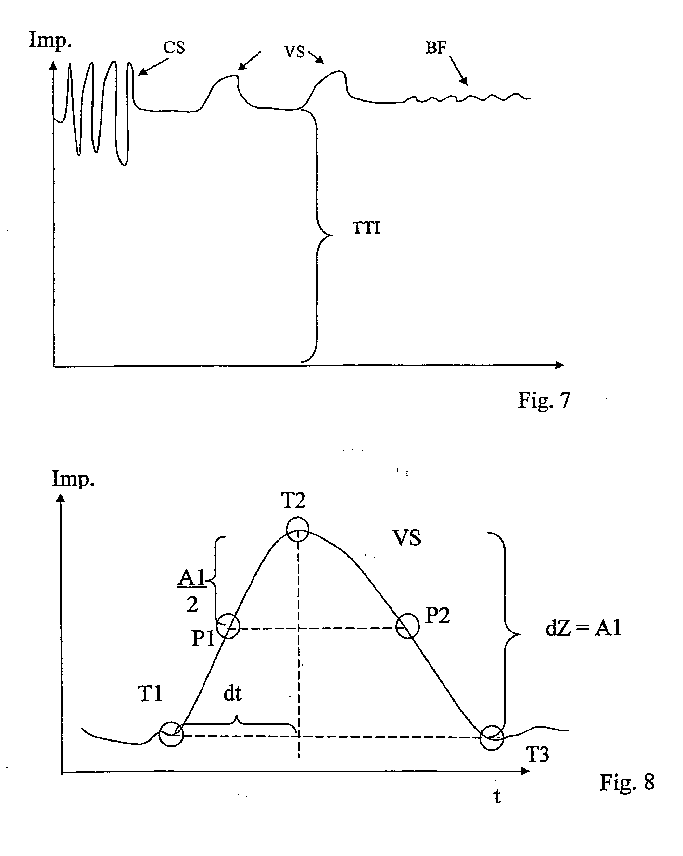

Also, the invention is described using only two electrodes, but more than two electrodes may also be used according to the invention, e.g. for more precisely measuring of impedance at a chosen depth, e.g. for reducing disturbances from the skin interface. Referring again to figure 2 the adaptive filter method is implemented in the signal processing unit (SPU). Signal inputs are the impedance signal, the force, and acceleration signal from the CCS. The impedance signal according to figure 7 comprises one signal component which represents the trans thoracic impedance (TTI), one further signal component which variations represent chest rise due to ventilations (VS), then again one signal component which variations represent chest movement due to chest compressions (CS), and potentially one component which variations represent blood flow (BF).

First step in signal processing is to remove baseline offset (like the TTI component) and high frequency components (like BF components) from impedance signal, the force signal and the acceleration signal. Second step is to remove the chest compression component CS (artifact) from the filtered impedance signal using the digital adoptive filter. This digital adaptive filter estimates the chest compression component using the force signal and the acceleration signal, and subtracts this estimate from the filtered impedance signal to output the ventilation signal. (Equivalent to how CPR artifacts are removed from ECG, in EP1079310.)

Based on the output ventilation signal, potential ventilations are detected and later classified as ventilations or not ventilations. According to Fig 8, a potential ventilation event is detected by searching for a positive impedance changes in the filtered ventilation signal having a distinct start point T1 and a top point T2. In addition to the start and top point, two more points are selected: P1, which is located half way between the start and top point on the rising edge and P2 half way down on the falling edge. The time interval from T1 to T2 is called dt, and the difference in amplitude between T2 and T1 is called dZ. Time interval from P1 to T2 is called P1T2, and the time interval from T2 to P2 is called T2P2. Real patient data, where the ventilations have been identified and manually annotated by an expert, has been used to identify the patient ventilation characteristics, based on the T1, T2, P1 and P2 points. The potential ventilation which is automatically detected is classified as ventilation or not as ventilation based on the identified ventilation characteristics. For instance, the system compare the actual value of dt and dz, P1T2, T2P2 and the ratio dz/dt with the acceptable ranges identified imperically from real patient data. If all selected criteria are met, the present impedance change is classified as a ventilation, where dt express inflation time and dz express the amplitude. When several ventilations has been detected, the system calculates ventilation rate by averaging the time intervals between successive T2 time points. This can be done because the impedance change is highly correlated with the volume of air delivered.

Time for which intra-thoracic pressure is elevated is estimated taking the time T1' defined from where the impedance waveform amplitude is 90% below the peak amplitude and rising, to the time T3' where the impedance waveform is 90% below the peak amplitude and falling. Tidal volume is estimated based on the relationship between impedance change dZ and ventilated volume in ml/kg. Experiments suggest that the relationship is approximately 0.17 ohm/ml/kg for patients in cardiac arrest. However, since the relationship is affected by both patient individuals and also where the electrode pads are placed, the relationship is not precise. For that reason, it can only be used to differentiate between smaller tidal volumes and larger tidal volumes.

Intra-thoracic pressure increases with each chest compression. For this reason, it can be assumed that the airway pressure during ventilation and simultaneous chest compressions is higher than the airway pressure found without ongoing chest compressions. When pressure increased, and temperature is near constant, volume is reduced. In other words, during chest compressions, one delivered tidal volume will be smaller than the expired tidal volume, because of the pressure difference. Laboratory experiments found that the impedance response with fixed volume ventilation was 20% lower when a significant weight was placed on the patient's chest vs without weight. Hence to improve precision, the estimated tidal volume shall be corrected by a compensation factor when chest compressions are ongoing. The compensation factor can be fixed for simplicity, or made as a function of the applied chest compression force.

Referring now to figure 6 the SPU is also arranged to characterize chest compressions from the acceleration and force signal Fo. The force signal is compared with a reference value V1 (for the time set to 2 kg above the previous compression's minimum force value, or 2 kg above zero, if pause in compressions) for the purpose of determining the point in time where the compression has started, Ts, and where the compression has ended, Te. The two time points Ts and Te is then empirically adjusted to values Ts' and Te' before they are used as starting points and end points respectively for double integration of an acceleration signal in order to estimate chest compression movements. The force signal Fo is compared to a reference value V2 (for the time set to 3 kg) in the interval between Te' and the following compression's Ts', which is the time interval between two successive compressions. If the measured force signal exceeds the reference value V2, an incomplete pressure release event is issued. If the calculated depth of compression is less than the guidelines recommendation, and at the same time the corresponding peak value of the force signal exceeds a reference value V3, (for the time set to 50kg), corrective verbal feedback on insufficient depth is withheld. If the calculated depth of compression is greater than the guidelines recommendation, and at the same time the corresponding peak value of the force signal is less than a reference value V4, corrective verbal feedback on excessive depth is withheld.

Alternatively, said force criteria to withhold feedback can be replaced or supplemented by alternative criteria, for instance based on the power or work used by the rescuer to compress the chest. Power can be easily calculated by multiplying the applied force with the speed of the chest. The applied work can be determined by integration of the applied power. Both the average and peak power during a compression can be used as criteria for withholding feedback. For instance feedback on too low compression depth can be withheld if the average power during one or more compressions exceeds a certain threshold, for instance in the range of 2 -10 W. Such criteria can either be based on the net or on the gross work or power applied during the compression. The net average work (work delivered to the chest during compression - the work received from the chest during release) is directly related to the energy added to the chest during a compression. A significant part of this energy is consumed in viscous work to drive blood through the cardiovascular system during compressions

With the availability of force and estimated depth of compression, one alternative method to characterize incomplete release is to estimate the offset in depth caused by the incomplete release

This can be accomplished by calculating the stiffness of the chest near the point of minimum compression depth, and then calculating the ratio of force to stiffness to obtain depth. The stiffness of the chest can for instance be estimated from the measured force-depth curve. To estimate stiffness, the viscous component of the force must first be subtracted from the total force, for instance by assuming a viscous damping parameter being dependent of depth and giving rise to a damping force proportional to the speed of the chest (F_v=µv, where F_v is the viscous force, µ is the damping parameter and v the speed of the chest). The value of µ at different depths can be estimated by observing the width of the hysteresis loop in the force-depth curve. In a similar manner, the friction forces, being dependent only on the direction of movment and not the speed, may be subtracted. The moving mass of the chest, giving rise to inertial forces, can also be estimated by correlating acceleration and force, for instance near the minimum or maximum points of compression where the viscous force is low.

Some patients are treated while still in bed. The compliance of the mattress will cause movement in both the chest and the mattress. With the availability of force and depth, it is possible to characterize the relationship between force and depth for situations where CPR is done in a bed vs on a non-compliant surface. The characteristic force/depth relationship for compliant surfaces, when detected, is used to invalidate the depth information which in turn avoid misleading feedback. And to issue a warning signal.

The presence of a mattress beneath the patient can be assessed by combining several indicators calculated from the force and depth data. Examples of such indicators can for instance be the moving mass (which will be higher if the whole body moves on a mattress), the damping parameter (which is likely to be more constant with depth for a mattress), the compression depth (which is likely to be higher) and the stiffness (which is likely to be lower). These indicators can either be used alone or combined, for instance using a multivariate or neural network model, for instance trained on actual measurement data, to indicate the presence of a mattress.

One alternative solution to deal with CPR in beds is to arrange the system with a separate reference accelerometer SRA. This reference accelerometer is located in a pad which is to be placed under the patient's back. With this arrangement, the SPU can subtract the acceleration under the back from the acceleration seen by the CCS, equivalent to how a reference accelerometer is used in EP1057451 to compensate for motion when the chest compression is performed in a moving vehicle or similar.

All measured signals, such as of force signal, acceleration signal, depth signal, impedance signal, events, etc may of course be recorded for later reference and analysis, and possibly for adjusting the models and calculations performed in the SPU. The signals may also be communicated, e.g. by cable radio transmission of an associated mobile phone directly to an external unit for monitoring and additional feedback.

## Claims

1. Ventilation monitoring system for monitoring the ventilation of a patient, comprising:
at least two electrodes adapted to be positioned on a patient's chest coupled to impedance measuring means for measuring the impedance in the patient's chest, said impedance measurements being performed by applying an alternating current or voltage to said electrodes and measuring the impedance between them,
measuring means for measuring at least one chosen parameter affecting the measured impedance in a per se known way, said system also comprising a force sensor measuring the force applied to the chest, and
a processing unit for providing a signal indicative of the ventilation of the patient from said impedance measurement, said processing unit also being adapted to remove the effects of said measured chosen parameters from said ventilation signal.

2. System according to claim 1 also comprising a movement sensor adapted to be positioned on the patient's chest for measuring the movement of the patient's chest and coupled to said processing unit for calculating and applying an adaptive filter to said measured impedance, said filter being adapted to remove the effects of the movements from said impedance signal.

3. System according to claim 2, wherein said force sensor e.g. during CPR, is coupled to said processing unit, said adaptive filter also including the signals from said force sensor.

4. System according to claim 2, also comprising an acceleration sensor measuring the acceleration applied to a certain point on the chest, e.g. during CPR, and coupled to said processing unit, said adaptive filter also including the signals from said acceleration sensor.

5. System according to claim 2, also comprising a second movement sensor coupled to said processing unit and positioned on the patient's back for measuring the movements of the patient, said processing unit providing a difference signal indicative of the chest movement relative to the back, and thus the part of the movement affecting the impedance signal.

6. System according to claim 1 wherein the force sensor is coupled to said processing unit, said processing unit being adapted to monitor the minimum peak values of said force signal so as to whether the pressure has been completely released from the chest.

## Patentansprüche

1. Ventilationsüberwachungssystem zur Überwachung der Ventilation eines Patienten, umfassend:
mindestens zwei Elektroden, die dafür eingerichtet sind, auf der Brust eines Patienten positioniert zu werden, und mit Impedanzmessmitteln zum Messen der Impedanz in der Brust des Patienten gekoppelt sind, wobei die Impedanzmessungen durchgeführt werden, indem ein Wechselstrom oder eine Wechselspannung an die Elektroden angelegt wird und die Impedanz wischen ihnen gemessen wird,
Messmittel zum Messen mindestens eines gewählten Parameters, der die gemessene Impedanz beeinflusst, in einer an sich bekannten Art und Weise, wobei das System außerdem einen Kraftsensor umfasst, der die an die Brust angelegte Kraft misst, und
eine Verarbeitungseinheit zum Bereitstellen eines Signals, das für die Ventilation des Patienten indikativ ist, aus der Impedanzmessung, wobei die Verarbeitungseinheit außerdem dafür eingerichtet ist, die Auswirkungen der gemessenen gewählten Parameter aus dem Ventilationssignal zu entfernen.

2. System nach Anspruch 1, das außerdem einen Bewegungssensor umfasst, der dafür eingerichtet ist, zum Messen der Bewegung der Brust des Patienten auf der Brust des Patienten positioniert zu werden, und zum Berechnen eines adaptiven Filters und Anwenden desselben auf die gemessene Impedanz mit der Verarbeitungseinheit gekoppelt ist, wobei das Filter dafür eingerichtet ist, die Auswirkungen der Bewegungen aus dem Impedanzsignal zu entfernen.

3. System nach Anspruch 2, wobei der Kraftsensor z.B. während einer kardiopulmonalen Reanimation (CPR) mit der Verarbeitungseinheit gekoppelt ist, wobei das adaptive Filter außerdem die Signale von dem Kraftsensor enthält.

4. System nach Anspruch 2, das außerdem einen Beschleunigungssensor umfasst, der die an einen gewissen Punkt auf der Brust angelegte Beschleunigung misst, z.B. während einer CPR, und mit der Verarbeitungseinheit gekoppelt ist, wobei das adaptive Filter außerdem die Signale von dem Beschleunigungssensor enthält.

5. System nach Anspruch 2, das außerdem einen zweiten Bewegungssensor umfasst, der mit der Verarbeitungseinheit gekoppelt ist und zum Messen der Bewegungen des Patienten auf dem Rücken des Patienten positioniert ist, wobei die Verarbeitungseinheit ein Differenzsignal liefert, das für die Bewegung der Brust relativ zum Rücken und daher für den Teil der Bewegung, der das Impedanzsignal beeinflusst, indikativ ist.

6. System nach Anspruch 1, wobei der Kraftsensor mit der Verarbeitungseinheit gekoppelt ist, wobei die Verarbeitungseinheit dafür eingerichtet ist, die minimalen Spitzenwerte des Kraftsignals zu überwachen, um zu bestimmen, ob die Brust vollständig von dem Druck befreit worden ist.

## Revendications

1. Système de surveillance de ventilation pour surveiller la ventilation d'un patient, comprenant :
au moins deux électrodes adaptées pour être positionnées sur la poitrine d'un patient et couplées à des moyens de mesure d'impédance pour mesurer l'impédance sur la poitrine du patient, lesdites mesures d'impédance étant effectuées en appliquant un courant alternatif ou une tension sur lesdites électrodes, et en mesurant l'impédance entre celles-ci,
des moyens de mesure pour mesurer au moins un paramètre choisi affectant l'impédance mesurée d'une manière connue en soi, ledit système comprenant également un capteur de force mesurant la force appliquée sur la poitrine, et
une unité de traitement pour fournir un signal indiquant la ventilation du patient à partir de ladite mesure d'impédance, ladite unité de traitement étant également adaptée pour supprimer dudit signal de ventilation les effets desdits paramètres choisis mesurés.

2. Système selon la revendication 1, comprenant également un capteur de mouvement adapté pour être positionné sur la poitrine du patient afin de mesurer le mouvement de la poitrine du patient, et couplé à ladite unité de traitement pour calculer et appliquer un filtre adaptatif à ladite impédance mesurée, ledit filtre étant adapté pour supprimer dudit signal d'impédance les effets des mouvements.

3. Système selon la revendication 2, dans lequel ledit capteur de force, par exemple pendant une réanimation cardiorespiratoire, est couplé à ladite unité de traitement, ledit filtre adaptatif comprenant également des signaux provenant dudit capteur de force.

4. Système selon la revendication 2, comprenant également un capteur d'accélération mesurant l'accélération appliquée à un certain point sur la poitrine, par exemple pendant une réanimation cardiorespiratoire, et couplé à ladite unité de traitement, ledit filtre adaptatif comprenant également les signaux provenant dudit capteur l'accélération.

5. Système selon la revendication 2, comprenant également un second capteur de mouvement couplé à ladite unité de traitement et positionné sur le dos du patient pour mesurer les mouvements du patient, ladite unité de traitement fournissant un signal de différence indiquant le mouvement de la poitrine par rapport au dos, et ainsi la partie du mouvement affectant le signal d'impédance.

6. Système selon la revendication 1, dans lequel le capteur de force est couplé à ladite unité de traitement, ladite unité de traitement étant adaptée pour surveiller les valeurs de crête minimum dudit signal de force de manière à savoir si la pression a été complètement relâchée sur la poitrine.
